# FASCICULE DE BREVET EUROPEEN

(11) **EP 1 699 346 B1**
(45) Date de publication et mention de la délivrance du brevet: **06.04.2016**
(21) Numéro de dépôt: 04816382.8
(22) Date de dépôt: 14.12.2004
(51) Int. Cl.: A61B 3/02, G02C 5/00, G02C 13/00

(54) **MESURE DU COMPORTEMENT D'UN PORTEUR DE LENTILLES OPHTALMIQUES**
MESSUNG DES VERHALTENS EINES KONTAKTLINSENTRÄGERS
MEASUREMENT OF A CONTACT LENS WEARER BEHAVIOUR

(30) Priorité: 23.12.2003 FR 0315374
(43) Date de publication de la demande: 13.09.2006
(73) Titulaire: ESSILOR INTERNATIONAL (Compagnie Générale d'Optique), 94220 Charenton le Pont (FR)
(72) Inventeur: BONNIN, Thierry, F-94220 Charenton le Pont (FR)
(74) Mandataire: Cabinet Plasseraud
(86) Numéro de dépôt international: PCT/FR2004/003215
(87) Numéro de publication internationale: WO 2005/070284

(56) Documents cités:
- FR-A- 1 017 134
- FR-A- 1 073 615
- GB-A- 121 837
- GB-A- 286 932
- US-A- 3 211 047
- US-A- 5 489 953
- US-A1- 2003 107 707

## Description

La présente invention concerne le comportement d'un porteur de lentilles ophtalmiques. Plus spécifiquement, elle concerne les systèmes et procédés permettant de mesurer ou caractériser le comportement d'un porteur de lentilles ophtalmiques.

Les lentilles ophtalmiques (ou verres) sont fournies à un porteur dans une monture, de sorte que les lentilles sont à distance des yeux du porteur. Pour regarder un point dans l'espace, le porteur peut déplacer la tête, effectuer un mouvement des yeux, ou encore combiner un mouvement de la tête et des yeux. Par exemple, pour la lecture d'un livre de taille habituelle, la tête de l'utilisateur reste le plus souvent fixe et seuls les yeux se déplacent pour suivre les lignes du texte; en revanche, un porteur de lentille conduisant une voiture automobile utilise généralement une combinaison des mouvements de la tête et des yeux.

Pour la prescription de lunettes à lentilles multifocales progressives, on envisage depuis de nombreuses années des méthodes de prescription personnalisée visant à calculer ou sélectionner individuellement la conception de lentille progressif - le "design" progressif selon l'appellation courante - qui s'adapte le mieux au comportement dynamique de chaque porteur, notamment en termes de mouvements de la tête et des yeux.

Un test individuel permet d'attribuer à chaque porteur un coefficient quantifiant sa propension à bouger plutôt la tête ou plutôt les yeux ("Head mover / Eye mover", en langue anglaise, soit en traduction littérale, "bougeur de tête / bougeur d'yeux"). Ce coefficient comportemental préside au calcul du design le mieux adapté. Ainsi, par exemple, pour un porteur qui a tendance à bouger les yeux davantage que la tête, choisira-t-on un design présentant un champ de vision nette assez large. Au contraire, pour un porteur qui a tendance à bouger davantage la tête et moins les yeux, on privilégiera la douceur périphérique pour éviter les effets de tangage. On pourra également jouer sur la longueur de progression.

En pratique, une étape critique et délicate à mettre en oeuvre, tant d'un point de vue technique que commercial, s'avère être le test individuel permettant de mesurer la tendance comportementale de chaque porteur. Le besoin se fait en particulier ressentir d'un dispositif permettant de réaliser de manière à la fois simple, fiable et répétitive la mesure des mouvements de la tête et des yeux du porteur à équiper.

Il existe certes déjà sur le marché des dispositifs permettant cette mesure. Ces dispositifs comportent principalement :
- une partie fixe comprenant plusieurs sources lumineuses ponctuelles aptes à être activés alternativement et réparties suivant une ligne horizontale, l'une centrale disposée face au porteur et d'autres latérales disposées de part et d'autre de la source centrale à des emplacement définis, et
- une partie mobile placée sur la tête du porteur à la façon d'un couvre chef et dont la position angulaire autour de la verticale est repérée par rapport à la partie fixe.

Lorsqu'une source latérale est éclairée, le porteur regarde celle-ci en tournant plus ou moins la tête conformément à son habitude comportementale. L'angle de rotation de la tête est alors mesuré et est représentatif de la propension du porteur à tourner davantage la tête ou davantage les yeux lorsque son regard est attiré latéralement.

Un exemple d'un dispositif de ce type et de sa mise en oeuvre est décrit dans l'article Head movement propensity, James H. Fuller, Experimental Brain Research 1992, pp 152-164. Plus récemment, on a proposé l'utilisation, dans un procédé de mesure analogue, de capteurs modernes sans fils du type de ceux commercialisés sous la marque 3SPACE® FASTRAK® par la société Polhémus Incorporated Colchester, Vermont U.S.A. et décrits dans la brochure «User's manual Revision F», OPM3609-002C, Novembre 1993, ou toute édition plus récente de celle-ci.

Ces dispositifs existants donnent satisfaction sur le plan technique, mais sont en pratique trop coûteux et complexes ou peu commode à mettre en oeuvre pour une diffusion à grande échelle auprès des opticiens. Le besoin se fait donc ressentir d'un dispositif de mesure plus simple et moins coûteux.

Plus généralement, pour la conception de lentilles ophtalmiques, il peut être utile de caractériser le comportement d'un porteur ou d'une population donnée. Il existe donc un besoin de systèmes et de procédés permettant de mesurer ou caractériser le comportement d'un porteur de lentilles ophtalmiques.

Dans un mode de réalisation, l'invention propose donc Un procédé de mesure du comportement de la tête et des yeux d'un porteur lorsqu'il regarde une cible, comprenant les étapes de :
- fourniture d'une cible et équipement du porteur avec une lentille présentant au moins deux zones, la vision de la cible à travers une zone de la lentille différant de la vision de la cible à travers une zone voisine de la lentille;
- lorsque le porteur regarde la cible, détermination de la zone de la lentille à travers laquelle le porteur voit la cible en fonction de la vision qu'a le porteur de la cible, et
- calcul du mouvement de la tête et des yeux du porteur en fonction de la zone déterminée.

Dans un mode de mise en oeuvre du procédé, l'étape de calcul comprend:
- une étape de calcul du mouvement des yeux du porteur par rapport à la tête en fonction de la zone déterminée, et
- une étape de calcul du mouvement de la tête du porteur par rapport au buste en fonction de la position de la cible et du mouvement des yeux du porteur.

Cette étape de détermination peut s'effectuer en masquant un des yeux du porteur.

L'invention propose d'utiliser une lentille ophtalmique non correctrice présentant au moins deux zones, la vision à travers une zone de la lentille différant de la vision à travers une zone voisine de la lentille.

Les zones de la lentille peuvent s'étendre verticalement, ou présenter des limites parallèles.On peut aussi prévoir qu'une zone de la lentille s'étend sur une plage angulaire de 8 à 10° dans les conditions du porté moyennes.

Deux zones voisines de la lentille peuvent présenter des couleurs différentes et/ou être séparées par une bande noire. On peut encore prévoir que la zone médiane de la lentille est transparente.

L'invention propose enfin d'utiliser une lentille ophtalmique non correctrice présentant au moins deux zones et d'une cible, la perception de la cible à travers une zone de la lentille différant de la vision à travers une zone voisine de la lentille.

Une zone de la lentille peut filtrer la lumière suivant une polarisation différente de la polarisation d'une zone voisine.

D'autres avantages et caractéristiques de l'invention apparaîtront à la lecture de la description qui suit des modes de réalisation de l'invention, donnés à titre d'exemple et en référence aux dessins qui montrent:
- figures 1 à 3, un schéma en vue de dessus, en coupe, de la tête et des yeux d'un porteur, dans diverses configurations;
- figure 4, une vue de face d'une lentille selon l'invention.

L'invention est décrite dans la suite dans une application à la mesure du mouvement de la tête et des yeux autour d'axes verticaux, autrement dit pour un mouvement de rotation de la tête par rapport au buste de droite à gauche et pour un mouvement de rotation des yeux par rapport à la tête de gauche à droite.

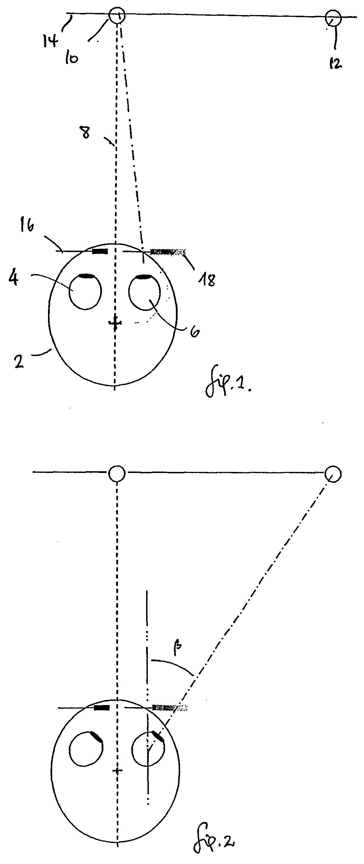

Les figures 1 à 3 montrent des schémas en vue de dessus, en coupe, de la tête et des yeux d'un porteur, dans diverses configurations. On a représenté sur ces figures la tête 2 du porteur, qui est susceptible de tourner, par rapport au corps du porteur, autour d'un axe de rotation vertical; cet axe est donc perpendiculaire à la surface de la feuille. Sont aussi représentés de façon schématique les yeux gauche 4 et droit 6 du porteur. Chaque oeil est adapté à tourner par rapport à la tête, autour du centre de rotation de l'oeil, qui n'est pas représenté sur les figures. Le trait interrompu 8 représente la direction principale de vision, autrement dit le plan vertical s'étendant devant la tête du porteur; ce plan est le plan médiateur des deux yeux 4, 6 du porteur. La référence 10 désigne une cible ou point de l'espace objet situé devant le porteur, à une distance donnée du porteur; cette distance est par exemple mesurée par rapport à l'axe de rotation de la tête, matérialisé par une croix sur les figures. La référence 12 désigne une autre cible - un autre point de l'espace objet; ce point est situé dans un plan 14 vertical, perpendiculaire à la direction principale de vision et passant par le point 10; l'angle entre les points 10 et 12, vu depuis le porteur, est de l'ordre de 40°. Le trait mixte sur les figures 1 et 2 montre la direction du regard pour l'oeil droit. Sont encore représentées sur les figures 1 à 3 les lentilles gauche 16 et droite 18 portées par le porteur.

La figure 1 montre le porteur, dans la configuration où il regarde droit devant lui. L'angle de rotation de la tête par rapport au buste est nul et l'angle de rotation des yeux par rapport à la tête est lui aussi nul. Ainsi, la direction principale de vision est confondue avec le plan s'étendant depuis le buste du porteur vers le point 10. La direction du regard est parallèle à la direction principale de vision.

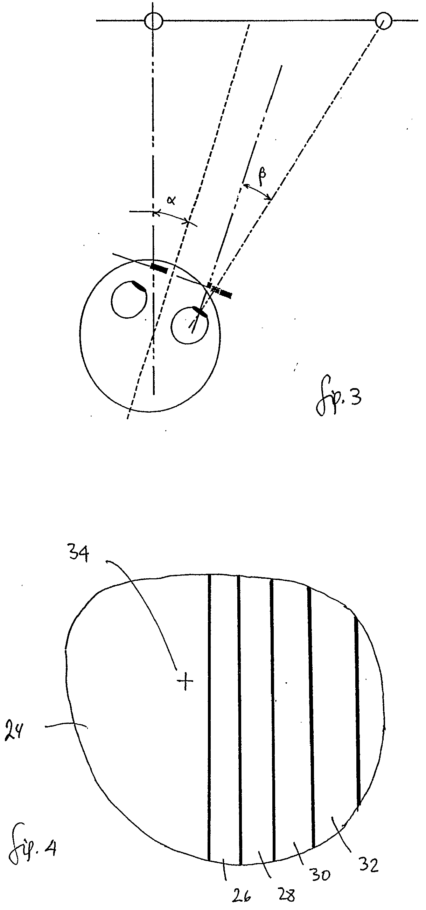

Les figures 2 et 3 montrent des configurations où le porteur regarde la cible ou point 12. Dans la configuration de la figure 2, le porteur ne tourne pas la tête par rapport au buste, mais tourne simplement les yeux par rapport à la tête; l'angle α de rotation de la tête par rapport au buste reste nul, comme dans la configuration de la figure 1. En revanche, l'angle β de rotation des yeux par rapport à la tête est de l'ordre de 40°. Dans la configuration de la figure 3, le porteur tourne la tête par rapport au buste et tourne aussi les yeux par rapport à la tête. Le porteur regarde donc la cible 12 par une combinaison d'un mouvement de la tête par rapport au buste, sur un angle α d'environ 20° et d'un mouvement des yeux par rapport à la tête, sur un angle β de l'ordre de 20°.

Les figures 1 à 3 illustrent donc différents comportements du porteur pour regarder la cible 12. L'invention a pour objet de mesurer ou caractériser le comportement, c'est-à-dire de déterminer comment le porteur combine les mouvements de la tête et des yeux pour regarder un point de l'espace objet qui ne se trouve pas directement devant lui.

Dans un mode de réalisation, l'invention propose d'utiliser pour mesurer le comportement du porteur une lentille présentant des zones de perception différentes, comme représenté à la figure 4. Cette figure montre une représentation schématique de la lentille 18 de la figure 1, en vue de face; dans l'exemple de la figure 4, on mesure le comportement du porteur pour des mouvements de la tête et des yeux autour d'axes verticaux. Comme le montre la figure, la lentille est séparée en différentes zones 24, 26, 28, 30 et 32, verticales - c'est-à-dire sensiblement perpendiculaires à la direction du mouvement mesuré. Ces zones ou franges sont au nombre de cinq dans l'exemple, pour les raisons discutées plus bas. Ces franges ont pour fonction de modifier la perception que le porteur a d'une cible, en fonction de la zone à travers laquelle il regarde la cible. Différentes solutions structurelles sont possibles pour assurer cette fonction. Dans un premier exemple, les franges présentent des couleurs différentes; on peut par exemple utiliser un traitement de surface de la lentille ou un traitement de la lentille dans la masse pour lui impartir des couleurs; le traitement de surface peut comprendre l'application d'un film filtrant sur une surface de la lentille, l'application d'une peinture de type vitrail, ou encore un dépôt par traitement sous vide. Le traitement dans la masse peut comprendre une coloration par trempage. On peut pour appliquer ces traitement procéder à un masquage partiel de la lentille. Ces couleurs différentes ont pour effet que le porteur perçoit différemment la cible selon la zone de la lentille à travers laquelle il regarde la cible. On peut par exemple utiliser pour les différentes zones les couleurs suivantes : rouge, vert, transparent, bleu, jaune. Ces différentes couleurs ont l'avantage d'être simples à percevoir.

En lieu et place des couleurs, ou en combinaison avec des couleurs, on peut aussi utiliser des gravures sur la lentille, par exemple des hachures présentant d'une zone à l'autre des directions différentes. Cette solution présente l'avantage de pouvoir appliquer la mesure à des porteurs présentant des défauts de vision des couleurs.

Dans un autre exemple, on peut utiliser pour les zones des filtres polarisants, avec des directions de polarisation différentes; on utilise alors plusieurs cibles proches, présentant des filtres analogues. Les cibles sont choisies proches, de sorte à être vues par le porteur à travers une même zone de la lentille; dans l'exemple de la figure 4, les cibles seraient les unes au dessus des autres. Selon la zone à travers laquelle le porteur regarde les cibles, il voit l'une ou l'autre des cibles. Par exemple, on pourrait utiliser pour les différentes zones des bandes polarisées avec une direction de polarisation verticale pour les première et quatrième bandes et une direction de polarisation horizontale pour les deuxième et cinquième bandes. La troisième bande est comme dans l'exemple précédent une bande transparente. La cible est formée de deux lampes superposées, de couleurs différentes, l'un présentant une polarisation verticale et l'autre une polarisation horizontale; selon la bande à travers laquelle il regarde la cible, le porteur voit donc, de la première bande à la cinquième: une lampe d'une première couleur, une lampe de l'autre couleur, les deux lampes, la lampe de la première couleur, puis enfin la lampe de la deuxième couleur. L'avantage par rapport à la solution précédente est que la lentille apparaît de teinte uniforme - comme un verre solaire, à l'exception de la bande centrale. Ceci peut être moins perturbant pour le porteur. On peut aussi utiliser une coloration (neutre en terme de polarisation) pour la bande centrale de sorte à fournir au porteur une lentille d'une teinte uniforme. Au lieu de cibles de couleurs différentes, on peut utiliser des cibles de formes différentes, permettant ainsi de mesurer le comportement d'utilisateur présentant des défauts de perception des couleurs. On peut encore combiner des cibles de formes différentes avec des polarisations variées.

On peut aussi combiner les différents exemples proposés pour définir sur ou dans la lentille les zones; plus généralement, on peut utiliser toute solution connue en soi pour modifier la perception ou la vision que le porteur a de la cible en fonction de la zone de la lentille à travers laquelle il regarde la ou les cibles.

On peut utiliser des couleurs ou des perceptions différentes pour chacune des zones; cette solution présente l'avantage que le porteur n'a pas besoin de compter le défilement des zones; on pourrait aussi n'utiliser que deux couleurs ou deux perceptions, en alternant les deux couleurs ou perceptions d'une zone à sa voisine.

La mesure s'effectue simplement en équipant le porteur d'une monture présentant la lentille de la figure 4 et en lui demandant de regarder différentes cibles. On peut à cette fin utiliser une monture d'essai permettant de fixer par clips la lentille; il est avantageux que les demi-écarts pupillaires soient réglables, pour amener une bande frontale - la bande transparente dans l'exemple précédent - en face de l'oeil du porteur. Le demi-écart pupillaire est mesuré avant le montage de la lentille à l'aide d'un pupillomètre.

Il est plus simple de procéder à une mesure oeil par oeil; en effet, ceci évite de devoir chercher des positions correspondantes des bandes de couleur sur les lentilles gauche et droite; bien que l'on puisse procéder successivement à une mesure sur l'oeil droit puis sur l'oeil gauche, il s'avère que le comportement du porteur est généralement identique pour un oeil et pour l'autre. Sur les figures 1 à 3, la mesure s'effectue pour l'oeil droit du porteur. En conséquence, comme le montrent les figures, la lentille gauche 16 est noircie et le porteur regarde le point 12 de l'espace objet par l'oeil droit. Les figures ne montrent donc que la direction du regard issu de l'oeil droit.

Dans la configuration de la figure 1, le porteur regarde droit devant lui. La tête est en position angulaire centrale et la direction du regard provenant de l'oeil droit traverse donc une zone de la lentille qui est la 1^{ère} zone utile en partant du côté nasal de la lentille. Il est avantageux que cette zone frontale soit transparente, pour permettre un recalage aisé de la vision.

Dans la configuration de la figure 2, le porteur regarde la cible 12; il ne tourne pas la tête (position centrale de tête), mais simplement les yeux. De la sorte, l'angle α est nul et l'angle P entre la direction du regard provenant de l'oeil droit et la direction principale de vision est de l'ordre de 40°. Le porteur regarde la cible à travers une zone de la lentille qui est la 5 ième zone utile en partant du côté nasal de la lentille.

Dans la configuration de la figure 3, le porteur regarde la cible 12, en tournant la tête et les yeux. De la sorte, l'angle α est de l'ordre de 20° l'angle β entre la direction du regard provenant de l'oeil droit et la direction principale de vision est de l'ordre de 20°. Le porteur regarde la cible à travers une zone de la lentille qui est la 3 ième zone utile en partant du côté nasal de la lentille.

Ainsi, en fonction du comportement du porteur - les figures 2 et 3 n'étant que deux exemples de comportement possibles - le porteur a une vision de la cible différente. Il est donc possible de simplement demander au porteur de regarder une cible et d'indiquer la perception qu'il a de la cible pour déterminer la zone de la lentille à travers laquelle le porteur regarde la cible. Dans l'exemple de zones présentant des couleurs différentes, le porteur de la figure 2 pourrait indiquer une couleur tandis que le porteur de la figure 3 pourrait indiquer une autre couleur.

On peut déterminer le comportement du porteur pour une cible située latéralement sur sa droite, comme dans l'exemple des figures 1 à 3; on peut aussi utiliser une cible située sur la gauche de l'utilisateur. Dans la pratique, il peut suffire de prévoir trois cibles, l'une droit devant l'utilisateur - pour amener l'utilisateur dans la configuration de la figure 1 - et les deux autres de part et d'autre de l'utilisateur, à des angles de ±40°. On notera que la position des cibles étant fixes, l'angle sous lequel le porteur voit les cibles dépend de la distance entre le porteur et le plan contenant les cibles; il est avantageux que cette distance soit mesurée de façon aussi précise que possible pour que la mesure soit précise.

La détermination de la zone de la lentille à travers laquelle le porteur regarde la cible permet de calculer l'angle de rotation des yeux par rapport à la tête - ou plus précisément, une plage d'angle de rotation des yeux par rapport à la tête. Cette plage angulaire dépend de la position des différentes zones sur la lentille et de la position de la lentille par rapport à l'oeil; cette position peut être mesurée pour le porteur, ou peut simplement être modélisée en utilisant un des modèles existants de l'oeil et des conditions du porté moyennes ou mesurées. On peut par exemple considérer le modèle de l'oeil proposé dans *Accommodation-dependent model of the human eye with aspherics*, R. Navarro, J. Santamaria and J. Bescos, Vol. 2, No 8 / August 1985, Opt. Soc. Am. A. Pour les conditions du porté, on peut considérer des valeurs moyennes, soit une distance de 27 mm entre le centre de rotation de l'oeil et le verre, une valeur de 12° pour l'angle pantoscopique et une valeur de 3 à 5° pour le galbe.

Dans l'exemple de la figure 4, on ménage cinq zones sur la lentille; chaque zone correspond à un secteur angulaire de l'ordre de 8° pour l'angle β de rotation des yeux par rapport à la tête. Pour avoir un secteur angulaire sensiblement constant d'une zone à l'autre, la largeur des zones sur la lentille croît depuis le point matérialisant la direction principale du regard vers le côté temporal ou le côté nasal. Dans l'exemple, on a représenté une lentille utilisable pour l'oeil droit, avec des bandes de couleur 26, 28, 30 et 32 du côté temporal, ce qui correspond à une cible sur la droite de l'utilisateur, comme sur la figure 1 ou sur la figure 3. Le reste de la lentille - sur la gauche de la bande 26 ou sur la droite de la bande 32, référencé 24 - est transparent ou n'est pas coloré. On a aussi représenté sur la figure le point 34 matérialisant la direction principale de vision. La lentille peut être montée sur une monture permettant un réglage simple de la hauteur et de l'écart pupillaire.

Pour faciliter la détermination de la zone de la lentille, il est avantageux de prévoir des bandes noires, entre les différentes zones de la lentille. Ces bandes permettent de bien marquer la différence entre les différentes zones et facilitent pour le porteur la sélection de la zone à travers laquelle il voit la cible. Une bande peut présenter une largeur de 1 mm sur la lentille, correspondant à une plage angulaire de l'ordre de 2°. Pour faciliter le recalage du porteur, il est avantageux que la zone centrale ou médiane de la lentille, qui correspond à une vision du porteur à l'infini, soit une zone transparente. Ceci permet un recalage plus aisé du porteur dans la configuration représentée à la figure 1. On peut utiliser des bandes noires autocollantes.

Une fois déterminé l'angle de rotation des yeux par rapport à la tête, il est possible, en fonction de la position de la cible, de déterminer la rotation de la tête. De fait, si la position latérale de la cible est connue, l'angle de rotation de la tête est déterminé comme l'angle nécessaire pour que la direction du regard - compte tenu de la rotation des yeux - passe par la cible. On comprend ainsi que dans la configuration de la figure 2, l'angle de rotation de la tête est nul, puisque l'angle de rotation des yeux - mesuré par la zone de la lentille - correspond sensiblement à l'angle sous lequel l'utilisateur voit la cible. En revanche, dans la configuration de la figure 3, l'angle de rotation des yeux est de l'ordre de 20°, alors que la cible est pour le porteur décalée vers la droite de 40°; on en déduit que l'angle de rotation de la tête est de l'ordre de 20°. En pratique, on peut assimiler les deux centres de rotation tête et oeil. Cela se justifie par la distance les séparant, qui est faible par rapport à la distance à la cible. Cette hypothèse fournit de bons résultats et évite une mesure de la distance entre les centres de rotation ainsi qu'un calcul plus complexe.

Des essais comparatifs montrent menés avec des lentilles selon l'invention et le système de l'état de la technique utilisant les capteurs de la société Polhémus montrent que le port des lentille décrites plus haut ne perturbe pas le comportement des porteurs.

La mesure est d'autant plus précise que la distance entre le centre de rotation de l'oeil et la lentille est connue avec précision et que la distance entre le porteur et la ou les cibles est connue avec précision. Il est difficile de déterminer la distance entre le centre de rotation de l'oeil et la lentille; on peut néanmoins adapter la position de la lentille de sorte à ce que la distance verre-oeil soit déterminée. Une autre solution consiste à procéder à une calibration du système de mesure en fixant la tête du porteur, en déplaçant la cible et en déterminant pour quelle position angulaire de la cible le porteur perçoit un changement de couleur de la cible.

Dans l'exemple de la figure 4, on a considéré cinq zones; on peut plus particulièrement utiliser des zones couvrant chacune une plage angulaire de 8 à 10°. La zone centrale couvrirait alors une vision de l'oeil dans une plage de ±4° à ±5° autour de la direction principale de vision. On peut alors utiliser des zones symétriques de part et d'autre de la zone centrale. Un choix de quatre couleurs permet de couvrir une plage angulaire de -45° à +45°, ce qui est suffisant pour la majorité des porteurs.

Bien entendu, l'invention n'est pas limitée aux exemples préférés donnés ci-dessus. On pourrait utiliser d'autres directions des zones de perception sur la lentille pour mesurer le comportement du porteur suivant d'autres axes de rotation que des axes verticaux; par exemple, des zones de perception horizontales permettent de mesurer le comportement du porteur lorsqu'il incline la tête de bas en haut et lorsqu'il baisse ou lève les yeux. Utiliser des zones de perception formant un damier sur la lentille permet de combiner des mesures suivant plusieurs directions. Dans les exemples, les zones de perception sont délimitées par des droites; on peut aussi, en fonction des comportements à mesurer, utiliser des zones de perception délimitées par des courbes. Tel pourrait par exemple être le cas, pour des mesures pour des porteurs habitués à utiliser des lentilles multifocales progressives.

L'invention n'est pas non plus limitée aux valeurs de nombres de zones et de largeurs de zones proposées dans les exemples de réalisation. On peut en fonction des besoins procéder à des mesures avec des zones plus ou moins nombreuses; il n'est pas non plus indispensable que les différentes zones ménagées sur la lentilles soient de tailles identiques; par exemple, on peut être intéressé à une mesure plus précise dans une plage angulaire donnée.

## Revendications

1. Un procédé de mesure du comportement de la tête et des yeux d'un porteur lorsqu'il regarde une cible, comprenant les étapes de :
- fourniture d'une cible (12) et équipement du porteur avec une lentille (18) présentant au moins deux zones (24, 26, 28, 30, 32), la vision de la cible à travers une zone de la lentille différant de la vision de la cible à travers une zone voisine de la lentille;
- lorsque le porteur regarde la cible, détermination de la zone de la lentille (18) à travers laquelle le porteur voit la cible en fonction de la vision qu'a le porteur de la cible, et
- calcul du mouvement de la tête et des yeux du porteur en fonction de la zone déterminée.

2. Le procédé de la revendication 1, **caractérisé en ce que** l'étape de calcul comprend:
- une étape de calcul du mouvement des yeux du porteur par rapport à la tête en fonction de la zone déterminée, et
- une étape de calcul du mouvement de la tête du porteur par rapport au buste en fonction de la position de la cible et du mouvement des yeux du porteur.

3. Le procédé de la revendication 1 ou 2, **caractérisé en ce que** l'étape de détermination s'effectue en masquant un des yeux du porteur.

4. Le procédé selon l'une des revendications 1 à 3, **caractérisé en ce que** les zones de la lentille (18) s'étendent verticalement.

5. Le procédé selon l'une des revendications 1 à 4, **caractérisé en ce que** les zones de la lentille (18) présentent des limites parallèles.

6. Le procédé selon l'une des revendications 1 à 5, **caractérisé en ce que** deux zones voisines de la lentille (18) présentent des couleurs différentes.

7. Le procédé selon l'une des revendications 1 à 6, **caractérisé en ce que** deux zones voisines de la lentille (18) sont séparées par une bande noire.

8. Le procédé selon l'une des revendications 1 à 7, **caractérisé en ce que** la zone médiane de la lentille (18) est transparente.

9. Le procédé selon l'une des revendications 1 à 8, **caractérisé en ce qu'**une zone de la lentille (18) filtre la lumière suivant une polarisation différente de la polarisation d'une zone voisine.

## Patentansprüche

1. Verfahren zum Messen des Verhaltens des Kopfs und der Augen eines Trägers, wenn er auf ein Ziel blickt, umfassend die folgenden Schritte:
- Bereitstellen eines Ziels (12) und Ausrüsten des Trägers mit einer Linse (18), die mindestens zwei Bereiche (24, 26, 28, 30, 32) aufweist, wobei die Sicht des Ziels durch einen Bereich der Linse von der Sicht des Ziels durch einen benachbarten Bereich der Linse verschieden ist,
- wenn der Träger auf das Ziel blickt, Bestimmen des Bereichs der Linse (18), durch den der Träger das Ziel sieht, in Abhängigkeit von der Sicht, die der Träger vom Ziel hat, und
- Berechnung der Bewegung des Kopfs und der Augen des Trägers in Abhängigkeit von dem bestimmten Bereich.

2. Verfahren nach Anspruch 1, **dadurch gekennzeichnet, dass** der Berechnungsschritt Folgendes umfasst:
- einen Schritt des Berechnens der Bewegung der Augen des Trägers bezüglich des Kopfs in Abhängigkeit von dem bestimmten Bereich und
- einen Schritt des Berechnens der Bewegung des Kopfs des Trägers bezüglich des Oberkörpers in Abhängigkeit von der Position des Ziels und der Bewegung der Augen des Trägers.

3. Verfahren nach Anspruch 1 oder 2, **dadurch gekennzeichnet, dass** der Bestimmungsschritt unter Abdeckung eines der Augen des Trägers erfolgt.

4. Verfahren nach einem der Ansprüche 1 bis 3, **dadurch gekennzeichnet, dass** sich die Bereiche der Linse (18) vertikal erstrecken.

5. Verfahren nach einem der Ansprüche 1 bis 4, **dadurch gekennzeichnet, dass** die Bereiche der Linse (18) parallele Grenzen aufweisen.

6. Verfahren nach einem der Ansprüche 1 bis 5, **dadurch gekennzeichnet, dass** zwei benachbarte Bereiche der Linse (18) verschiedene Farben haben.

7. Verfahren nach einem der Ansprüche 1 bis 6, **dadurch gekennzeichnet, dass** zwei benachbarte Bereiche der Linse (18) durch ein schwarzes Band getrennt sind.

8. Verfahren nach einem der Ansprüche 1 bis 7, **dadurch gekennzeichnet, dass** der mittlere Bereich der Linse (18) transparent ist.

9. Verfahren nach einem der Ansprüche 1 bis 8, **dadurch gekennzeichnet, dass** ein Bereich der Linse (18) das Licht nach einer Polarisation filtert, die von der Polarisation eines benachbarten Bereichs verschieden ist.

## Claims

1. Method for measuring the behaviour of the head and eyes of a wearer when he looks at a target, comprising steps of:
- providing a target (12) and equipping the wearer with a lens (18) having at least two zones (24, 26, 28, 30, 32), the vision of the target through one zone of the lens differing from the vision of the target through a neighbouring zone of the lens;
- when the wearer looks at the target, determining the zone of the lens (18) through which the wearer sees the target depending on the vision that the wearer has of the target; and
- calculating the movement of the head and eyes of the wearer depending on the determined zone.

2. Method according to Claim 1, **characterized in that** the calculating step comprises:
- a step of calculating the movement of the eyes of the wearer relative to their head, depending on the determined zone; and
- a step of calculating the movement of the head of the wearer relative to their torso, depending on the position of the target and on the movement of the eyes of the wearer.

3. Method of Claim 1 or 2, **characterized in that** the determining step is carried out while masking one of the eyes of the wearer.

4. Method according to one of Claims 1 to 3, **characterized in that** the zones of the lens (18) extend vertically.

5. Method according to one of Claims 1 to 4, **characterized in that** the zones of the lens (18) have parallel limits.

6. Method according to one of Claims 1 to 5, **characterized in that** two neighbouring zones of the lens (18) are different colours.

7. Method according to one of Claims 1 to 6, **characterized in that** two neighbouring zones of the lens (18) are separated by a black strip.

8. Method according to one of Claims 1 to 7, **characterized in that** the median zone of the lens (18) is transparent.

9. Method according to one of Claims 1 to 8, **characterized in that** a zone of the lens (18) filters light having a polarization different from the polarization of a neighbouring zone.
